# EUROPÄISCHE PATENTANMELDUNG

(11) **EP 1 800 671 A1**
(43) Veröffentlichungstag der Anmeldung: **27.06.2007**
(21) Anmeldenummer: 05090347.5
(22) Anmeldetag: 23.12.2005
(51) Int. Cl.: A61K 9/70

(54) **Verwendung filmbildender Haarpflegepolymere und diese Polymere enthaltenden pharmazeutischen Zubereitungen und Pflaster**

(71) Anmelder: Bayer Schering Pharma Aktiengesellschaft, 13353 Berlin (DE)
(72) Erfinder: Zurdo Schröder, Ines, 10437 Berlin (DE); Franke, Patrick, Dr., 14167 Berlin (DE); Bracht, Stefan, Dr., 16548 Berlin (DE)

(57) **Zusammenfassung**

Gegenstand der vorliegenden Anmeldung ist die Verwendung eines filmbildenden in Haarpflegemitteln Verwendung findenden Polymeres oder eines Gemisches verschiedener dieser Polymere in pharmazeutischen Zubereitungen zur dermalen oder transdermalen Applikation von Wirkstoffen, sowie Pflaster und pharmazeutische Zubereitungen enthaltend diese Haarpflegepolymere.

## Beschreibung

Die Erfindung betrifft die Verwendung filmbildender Haarpflegepolymere und pharmazeutische Zubereitungen enthaltend diese Haarpflegepolymere zur dermalen oder transdermalen Applikation von Wirkstoffen. Die pharmazeutischen Zubereitungen werden in flüssiger Form auf die Haut aufgetragen. Sie bilden dort durch Verdunstung des ebenfalls enthaltenen Lösungsmittels einen flexiblen, kosmetisch unauffälligen, gut haftenden, nicht-klebrigen Film, aus dem eine gesteuerte Wirkstoffabgabe in die Haut oder durch die Haut in den systemischen Kreislauf erfolgt.

Die menschliche Haut als Zielorgan der Arzneistoffapplikation wird gegenwärtig in zweifacher Hinsicht genutzt: Zum einen werden Wirkstoffe zur Behandlung verschiedener dermaler Erkrankungen wie Psoriasis oder Neurodermitis lokal appliziert. Zum anderen können Wirkstoffe zur Behandlung systemischer Erkrankungen und zur Hormonsubstitution transdermal verabreicht werden.

Bei der lokalen Applikation herrschen halbfeste Zubereitungen, wie Salben, Cremes oder Gele vor. Die transdermale Verabreichung findet zumeist über transdermale therapeutische Systeme, wie Matrix- oder Reservoirpflaster statt. In letzter Zeit gelangen auch halbfeste Produkte (hydroalkoholische Gele wie zum Beispiel Testogel^{®}) für eine transdermale Anwendung zum Einsatz.

In den am Markt befindlichen Gelformulierungen zur transdermalen Anwendung liegen die zu permeierenden Wirkstoffe als alkoholische Lösung vor. Sie werden mittels eines Polymers (zum Beispiel Polyacrylsäure) angedickt und in Form eines hydroalkoholischen Gels einmal täglich auf die Haut aufgebracht.

Da Alkohole in kurzer Zeit evaporieren, bleibt den Wirkstoffen ebenfalls nur ein kurzer Zeitraum, um in die Epidermis zu gelangen. Mehr als 90 % des Wirkstoffes gelangen daher nicht ins Blut, sondern verbleiben zum größten Teil auf der Hautoberfläche. Der rasche Verlust des Lösemittels führt zu einer Übersättigung, die häufig zur Auskristallisation von Wirkstoffen führt. Eine Penetration ist dann erheblich erschwert, da der Wirkstoff nur in gelöster Form in die Haut diffundieren kann.

Auf der Haut nicht fixierte Wirkstoffrückstände bergen die Gefahr eines Dosisverlustes sowie einer Kontamination der Kleidung oder anderer Personen.

Insbesondere bei der transdermalen Verabreichung werden häufig sehr große Applikationsflächen benötigt, um die erforderlichen Wirkstoffmengen durch die Haut zu transportieren. Dies erfordert hohe Gel-Volumina von mehreren Millilitern pro Einzeldosis. Von den Patienten wird dies in der Handhabung als unpraktisch und unangenehm empfunden. Schließlich ist eine Steuerung der Wirkstofffreisetzung über einen längeren Zeitraum bei klassischen Gel-Zubereitungen nicht möglich.

Im Gegensatz dazu ist bei transdermalen therapeutischen Systemen eine gesteuerte Wirkstofffreisetzung zwar möglich, jedoch ist in der Regel die Zahl der angebotenen Dosierungen aus vertriebslogistischen Gründen begrenzt. Eine individuelle, patientenbezogene Dosierung ist daher nur eingeschränkt möglich. Auch hinsichtlich der Kontaktfläche des transdermalen therapeutischen Systems zur Haut sind dem Dosierungsspielraum Grenzen gesetzt. Pflaster mit einer Größe jenseits der 20 cm² weisen durch ihre großflächige Hautfixierung einen geringen Tragekomfort auf. Weitere Nachteile sind durch die Adhäsivschicht des Pflasters hervorgerufene Hautirritationen, sowie kosmetische Beeinträchtigungen. Weiterhin erfordert die Herstellung transdermaler Pflaster aufwendige Geräte und Prozeduren.

Für viele der oben genannten Nachteile wurden bereits in der Vergangenheit Lösungsvorschläge angeboten. So wird in EP1150661 B1 ein topisches Spray enthaltend eine filmbildende Zusammensetzung und ein oder mehrere Wirkstoffe beansprucht. Dieses Spray enthält unter anderem filmbildende Polymere. Auf die Haut aufgetragen ergibt es einen flexiblen, gut haftenden atmungsaktiven Film.

Aufgabe der vorliegenden Erfindung ist es, alternative pharmazeutische Zubereitungen zu schaffen, die - auf die Haut aufgetragen - gegenüber bekannten Zubereitungen einen hinsichtlich der Wirkstoffpermeation verbesserten Film bilden.

Diese Aufgabe wurde erfindungsgemäß durch die Verwendung eines filmbildenden, gewöhnlicherweise in Haarpflegemitteln Verwendung findenden Polymeres oder eines Gemisches verschiedener dieser Polymere in pharmazeutischen Zubereitungen zur dermalen oder transdermalen Applikation von Wirkstoffen gelöst.

Es wurde gefunden, dass die in Haarpflegemitteln verwendeten Polymere überraschenderweise hinsichtlich Flexibilität, Haltbarkeit auf der Haut und Atmungsaktivität ähnlich gute Eigenschaften wie Zubereitungen mit bekannten Polymeren (wie zum Beispiel Eudragit^{®}) bei deutlich erhöhter Wirkstoffpermeation zeigen.

Hierzu werden die filmbildenden Haarpflegepolymere zusammen mit einem oder mehreren Wirkstoffen in einem geeigneten Lösungsmittel und unter Zusatz etwaiger Hilfsstoffe gelöst oder suspendiert und die so gebildete pharmazeutische Zubereitung flüssig auf die Haut aufgetragen.

Nach Verdunstung des Lösungsmittels auf der Haut bildet die pharmazeutische Zubereitung einen dünnen flexiblen unsichtbaren Film. Aus diesem Film können ein oder mehrere Wirkstoffe über das entstandene Polymerreservoir gesteuert an die Haut oder durch die Haut in den systemischen Kreislauf abgegeben werden. Die hier vorgeschlagene Applikationsmethode zeichnet sich durch eine einfache Applikation, zum Beispiel durch Versprühen oder Pinseln der pharmazeutischen Zubereitung, eine flexible Dosierung und eine steuerbare Wirkstofffreisetzung über einen längeren Zeitraum aus. Der entstandene Film zeigt gute Adhäsion auf der Haut bei geringem lrritationspotential und ein unauffälliges Aussehen. Durch die Fixierung des Wirkstoffes im Film auf der Haut wird ein Wirkstoffverlust durch Kontakt mit Kleidung oder eine Kontamination anderer Personen vermieden. Ein weiterer Vorteil der vorliegenden Erfindung ist, dass anders als bei der Herstellung transdermaler Pflaster keine aufwendigen Geräte oder Prozeduren benötigt werden. Die verschiedenen Bestandteile werden lediglich im Lösungsmittel gelöst oder suspendiert und abgefüllt. Das Aufbringen der Lösung kann unabhängig von der Beschaffenheit der Haut erfolgen. Im Gegensatz zum Pflaster, das auf eine haarlose, glatte Stelle aufgebracht werden sollte, stellen vorhandene Haare oder Falten kein Problem dar.

Während des Verdunstens des Lösungsmittels findet keine Rekristallisation der verwendeten Wirkstoffe statt. Vielmehr entsteht eine übersättigte Lösung, die eine hohe thermodynamische Aktivität zeigt. Dadurch wird die Permeation des Wirkstoffs in die Haut nachhaltig gefördert. Auch im angetrockneten Film kristallisiert der Wirkstoff nicht aus sondern liegt gelöst vor. Dies erlaubt eine Diffusion innerhalb des Films, so dass der Wirkstoff auch nach Trocknen des Films zur Grenzfläche diffundieren und an die Haut abgegeben werden kann. In die Formulierungen kann entweder ein einzelner Wirkstoff eingearbeitet werden oder mehrere Wirkstoffe (zum Beispiel Estrogene wie Ethinylestradiol in Kombination mit Gestagenen wie Levonorgestrel), die dann gemeinsam beziehungsweise parallel durch die Haut transportiert werden. Durch die Haarpflegepolymeren wird die Rekristallisation des Wirkstoffes vermieden und gleichzeitig thermodynamisch dessen Aufnahme durch die Haut begünstigt.

Zur erfindungsgemäßen Verwendung in pharmazeutischen Zubereitungen geeignete Haarpflegepolymerenklassen, sowie spezielle, besonders geeignete Vertreter können der folgenden Aufzählung entnommen werden. In der Aufzählung sind die Haarpflegepolymere nach ihren Markennamen aufgeführt. In der zugehörigen Klammer ist die Bezeichnung des Polymers und der jeweilige derzeitige Hersteller angegeben.

### Polyurethane:

- Luviset^{®} P.U.R. (Polyurethane-1, BASF),
- DynamX^{®} (Polyurethane-14 und AMP-Acrylat Copolymer, National Starch and Chemical),
- Avalure^{®} UR 405, Avalure^{®} UR 425 (Polyurethane-2, Noveon),
- Avalure^{®} UR 445 (Polyurethane-4, Noveon),
- Avalure^{®} UR 450 (Polypropylenglycol-17/ Isophoronediisocyanat/Dimethylpropionsäure Copolymer, Noveon)

### Monoalkylester des Poly (methylvinylether / Maleinsäureanhydrids oder der Maleinsäure):

- ADVANTAGE^{®} LC-55, ADVANTAGE^{®} LC-80 (Monoethylester des Copolymers von Methylvinylether und Maleinsäureanhydrid, ISP),
- GANTREZ^{®} ES-225, GANTREZ^{®} V-225, OMNIREZ^{®} 2000 (Ethylester des Polyvinylmethylether/Maleinsäureanhydrid Copolymers, ISP),
- GANTREZ^{®} ES-335I (Isopropylester des Polyvinylmethylether/Maleinsäureanhydrids, ISP),
- GANTREZ^{®} ES-425, GANTREZ^{®} SUPER A-425, GANTREZ^{®} ES-435 (Butylester des Polyvinylmethylether/Maleinsäureanhydrids, ISP)

### Acrylat/Alkylacrylamid Copolymere:

- AMPHOMER^{®} (Octylacrylamid/Acrylat/Butylaminoethylmethacrylat Copolymer, National Starch and Chemical),
- AMPHOMER^{®} 4961, AMPHOMER^{®} HC (Acrylat/Octylacrylamid Copolymer, National Starch and Chemical),
- AMPHOMER^{®} LV-71, AMPHOMER^{®} SOL (EUROPE), BALANCE^{®} 47 (Octylacrylamid/Acrylat/Butylaminoethylmethacrylat Copolymer, National Starch and Chemical),
- DERMACRYL^{®} 79, DERMACRYL^{®} LT, RESYN^{®} XP (Acrylat/Octylacrylamid Copolymer, National Starch and Chemical),
- Ultrahold^{®} 8, Ultrahold^{®} Strong (Acrylat/Ethylacrylat/N-tert-butylacrylamid Copolymere, BASF)

### Acrylat/Alkyl Acrylat/ Ethylaminoxidmethacrylat:

- Diaformer^{®} Z-711 N, Diaformer^{®} Z-712N, Diaformer^{®} Z-731 N (Acrylat / Laurylacrylat / Stearylacrylat/Ethylaminoxidmethacrylat Copolymer, Clariant),
- Diaformer^{®} Z-632N (Acrylat/Stearylacrylate/Ethylaminoxidmethacrylat Copolymer, Clariant),
- Diaformer^{®} Z-651 N (Acrylat/Laurylacrylat/Stearylacrylat/Ethylaminoxidmethacrylat Copolymer, Clariant)

### Acrylat/Itaconat Copolymere:

- STRUCTURE^{®} 2001 (Acrylat/Steareth-20 Itaconat Copolymer, National Starch and Chemical),
- STRUCTURE^{®} 3001 (Acrylat/Ceteth-20 Itaconat Copolymer, National Starch and Chemical),
- STRUCTURE^{®} PLUS (Acrylat/Aminoacrylat/C10-30 Alkyl PEG-20 Itaconat Copolymer, National Starch and Chemical)

### Vinylacetat/Crotonat Copolymere:

- Luviset^{®} CA 66 (Vinylacetat/Crotonat Copolymer, BASF),
- Luviset^{®} CAN (Vinylacetat/Crotonat/Vinylneodecanoat Copolymer, BASF),
- Luviset^{®} Clear (Vinylpyrrolidon/Methacrylamid/Vinylimidazol Copolymer, BASF),
- RESYN^{®} 28-1310 (Vinylacetate/Crotonat Copolymer, National Starch and Chemical),
- RESYN^{®} 28-2930 (Vinylacetat/Crotonat/Vinylneodecanoat Copolymer, National Starch and Chemical),
- Aristoflex^{®} A 60 (Vinylacetat/Crotonat Copolymer, Clariant)

### Vinylcaprolactam/Vinylpyrrolidon/Methacrylat Copolymere:

- ADVANTAGE^{®} HC-37, ADVANTAGE^{®} LC-A, ADVANTAGE^{®} S, GAFFIX^{®} VC-713 (Vinylcaprolactam/Vinylpyrrolidon/Dimethylaminoethylmethacrylat Copolymer, ISP),
- ADVANTAGE^{®} LC-E (Vinylcaprolactam/Vinylpyrrolidon/Dimethylaminoethylmethacrylat Copolymer und Laurylpyrrolidon, ISP),
- AQUAFLEX^{®} SF-40 (Vinylpyrrolidon/Vinylcaprolactam/Dimethylaminopropylmethacrylamid Copolymer, ISP)

### Vinylpyrrolidon/Methacrylamid Copolymere:

- STYLEZE^{®} CC-10 (Vinylpyrrolidon/Dimethylaminopropylmethacrylamid Copolymer, ISP)
- STYLEZE^{®} W-20 (Vinylpyrrolidon/Dimethylaminopropylmethacrylamid/Methacryloylaminopropyl lauryl dimethyl ammonium chlorid Copolymer, ISP),
- GAFQUAT^{®} HS100 (Copolymer von Vinylpyrrolidon und Methacrylamidopropyl trimethylammoniumchlorid, ISP)

Bevorzugt ist die Verwendung eines gegebenenfalls neutralisierten vollständig ausreagierten carboxylierten linearen Haarpflegepolyurethans, umfassend das Reaktionsprodukt aus
(1) einer oder mehreren 2,2-hydroxymethylsubstituierten Carbonsäure(n), dargestellt durch die Formel I in der R Wasserstoff oder C₁-C₂₀-Alkyl bedeutet, vorhanden in einer Gewichtsmenge, die ausreicht, um 0,35 - 2,25 Milliäquivalent Carboxylfunktionalität pro Gramm Polyurethan zu ergeben, 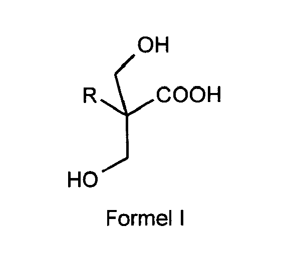
(2) 10 - 90 Gewichts-%, bezogen auf das Gewicht des Polyurethans, einer oder mehrerer organischen Verbindung(en), die jeweils nicht mehr als zwei aktive Wasserstoffatome aufweisen, und
(3) einem oder mehreren organischen Diisocyanat(en), vorhanden in einer Menge, die ausreicht, um mit den aktiven Wasserstoffatomen der 2,2-hydroxymethylsubstituierten Carbonsäure und der organischen Verbindungen, mit Ausnahme des Wasserstoffs an dem Carboxylat der 2,2-hydroxymethylsubstituierten Carbonsäure, zu reagieren.

Die 2,2-hydroxymethylsubstituierten Carbonsäuren werden dargestellt durch die Formel I 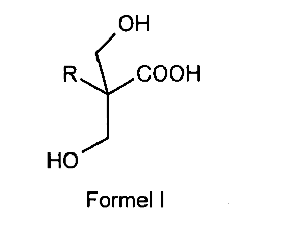 in der R Wasserstoff oder C₁-C₂₀-Alky1, vorzugsweise C₁-C₈-Alkyl bedeutet. Spezielle Beispiele umfassen 2,2-Di-(hydroxymethyl)essigsäure, 2,2-Di(hydroxymethyl)propionsäure, 2,2-Di(hydroxymethyl)buttersäure, 2,2-Di(hydroxymethyl)pentansäure und dergleichen. Die bevorzugte Säure ist 2,2-Di(hydroxymethyl)propionsäure. Die 2,2-hydroxymethylsubstituierten Carbonsäuren liegen in einer Menge vor, die 0,35 - 2,25, bevorzugt 0,5 - 1,85, Milliäquivalent Carboxylfunktionalität pro Gramm Polyurethan ergibt, und im allgemeinen ist das etwa 5 - 30 Gewichts-% des Polyurethanpolymers.

Die organischen Verbindungen, die mit Isocyanat reagieren und zur Herstellung der erfindungsgemäßen Polyurethanpolymere verwendet werden können, besitzen nicht mehr als zwei aktive Wasserstoffatome (bestimmt nach der Zerewitinoff-Methode). Die aktiven Wasserstoffatome sind normalerweise an Sauerstoff-, Stickstoff- oder Schwefelatomen gebunden. Diese Verbindungen haben ein Molekulargewicht von etwa 300 bis 20.000, bevorzugt etwa 500 bis 8.000. Vorzugsweise sind die Verbindungen linear, um ein Gelieren während der Polymerisation zu vermeiden, aber es können kleine Mengen an nicht-linearen Verbindungen unter der Voraussetzung eingesetzt werden, dass deren Einsatz kein Gelieren zur Folge hat. Die organischen Verbindungen liegen in einer Menge von 10 - 90 Gewichts-%, bevorzugt in einer Menge von 15 - 70 Gewichts-%, des Polyurethans vor.

Die bevorzugten organischen Verbindungen mit zwei aktiven Wasserstoffatomen sind die linearen bifunktionellen Polyethylen- und Polypropylenglykole, insbesondere die, welche handelsüblich sind und durch Reaktion von Ethylenoxid (oder Propylenoxid) mit Wasser, Ethylenglykol (oder Propylenglykol) oder Diethylenglykol (oder Dipropylenglykol) in Anwesenheit von Natriumhydroxid als Katalysator hergestellt werden. Diese Polyglykole besitzen Molekulargewichte von etwa 600 bis 20.000, bevorzugt etwa 1.000 bis 8.000. Es können Polyglykole mit homogenem Molekulargewicht oder ein Gemisch von Glykolen mit unterschiedlichem Molekulargewicht verwendet werden. Es ist auch möglich, kleine Mengen an zusätzlichen Alkylenoxiden durch Copolymerisation in das Polyglykol einzubauen.

Andere geeignete organische Verbindungen mit zwei aktiven Wasserstoffatomen sind die, die Hydroxyl-, Carboxyl-, Amino- oder Mercaptogruppen aufweisen. Unter ihnen sind Polyhydroxyverbindungen, wie Polyetherdiole, Polyesterdiole, Polyacetaldiole, Polyamiddiole, Polyesterpolyamiddiole, Poly(alkylenether)diole, Polythioetherdiole und Polycarbonatdiole, bevorzugt. Verbindungen mit zwei oder mehreren unterschiedlichen Gruppen innerhalb dieser Klassen, beispielsweise Aminoalkohole und Aminoalkohole mit zwei Aminogruppen und einer Hydroxylgruppe, können auch eingesetzt werden. Bevorzugt werden bifunktionelle Verbindungen verwendet, obwohl auch kleine Mengen an tri- (und mehr-) funktionellen Verbindungen eingesetzt werden können.

Geeignete Polyetherdiole sind beispielsweise die Kondensationsprodukte aus Ethylenoxid, Propylenoxid, Butylenoxid oder Tetrahydrofuran und deren Co-, Pfropf- oder Blockpolymerisationsprodukte, wie Ethylenmischoxid, Propylenoxidkondensate und die Pfropfpolymerisationsprodukte aus der Reaktion von unter hohem Druck stehenden Olefinen mit den erwähnten Alkylenoxidkondensaten. Geeignete Polyether werden durch Kondensation der genannten Alkylenoxide mit mehrwertigen Alkoholen, wie Ethylenglykol, 1,2-Propylenglykol und 1,4-Butandiol, hergestellt.

Geeignete Polyesterdiole, Polyesteramiddiole und Polyamiddiole sind vorzugsweise gesättigt und werden beispielsweise durch Reaktion von gesättigten oder ungesättigten Polycarbonsäuren mit gesättigten oder ungesättigten mehrwertigen Alkoholen, Diaminen oder Polyaminen erhalten. Für die Herstellung dieser Verbindungen geeignete Carbonsäuren umfassen beispielsweise Adipinsäure, Bernsteinsäure, Phthalsäure, Terephthalsäure und Maleinsäure. Für die Herstellung der Polyester geeignete mehrwertige Alkohole umfassen beispielsweise Ethylenglykol, 1,2-Propylenglykol, 1,4-Butandiol, Neopentylglykol und Hexandiol. Aminoalkohole, wie Ethanolamin, sind ebenfalls geeignet. Für die Herstellung der Polyesteramide und Polyamide geeignete Diamine sind beispielsweise Ethylendiamin und Hexamethylendiamin.

Geeignete Polyacetale können zum Beispiel aus 1,4-Butandiol oder Hexandiol und Formaldehyd hergestellt werden. Geeignete Polythioether können beispielsweise durch Kondensation von Thiodiglykol allein oder in Kombination mit anderen Glykolen, wie Ethylenglykol, 1,2-Propylenglykol, oder mit anderen Polyhydroxyverbindungen, wie zuvor offenbart, hergestellt werden. Polyhydroxyverbindungen, die bereits Harnstoff- oder Urethangruppen enthalten, und natürliche mehrwertige Alkohole, die weiter modifiziert sein können, beispielsweise Rizinusöl und Kohlenhydrate, können auch verwendet werden.

Bei der Herstellung des Polyurethanpolymers kann es wünschenswert sein, das Polymer, zusätzlich zur organischen Verbindung mit nicht mehr als zwei aktiven Wasserstoffatomen, die in vielen Fällen ein hohes Molekulargewicht besitzt, unter Verwendung einer organischen Verbindung mit einem niedrigeren Molekulargewicht, vorzugsweise von weniger als etwa 300 und mehr als 60, einer Kettenverlängerung zu unterziehen. Typische Kettenverlängerer umfassen gesättigte oder ungesättigte Glykole, wie Ethylenglykol, Diethylenglykol, Triethylenglykol und dergleichen; Aminoalkohole, wie Ethanolamin, Propanolamin, Butanolamin und dergleichen; mono- und dialkoxylierte aliphatische, cycloaliphatische, aromatische und heterocyclische primäre Amine, wie N-Methyldiethanolamin, N-Oleyldiethanolamin, N-Cyclohexyldiisopropanolamin, N,N-Dihydroxyethyl-p-toluidin, N,N-Dihydroxypropylnaphthylamin und dergleichen; Diamine, wie Ethylendiamin, Piperazin, N,N-Bis-gamma-aminopropyl-N-methylamin und dergleichen; Carbonsäuren, einschließlich aliphatischer, cycloaliphatischer, aromatischer und heterocyclischer Dicarbonsäuren, wie Oxalsäure, Malonsäure, Bernsteinsäure, Glutarsäure, Adipinsäure, Sebacinsäure, Terephthalsäure, Naphthalin-1,5-dicarbonsäure, Maleinsäure, Fumarsäure, Diglykolsäure, Chinolinsäure, Lutidinsäure und dergleichen; Aminocarbonsäuren, wie Glycin, alpha- und beta-Alanin, 6-Aminocapronsäure, 4-Aminobuttersäure, p-Aminobenzoesäure, 5-Aminonaphthoesäure und dergleichen. Die bevorzugten Kettenverlängerer sind aliphatische Diole.

Die organischen Polyisocyanate oder Gemische von Polyisocyanaten, die man mit der organischen Verbindung reagieren läßt, sind aliphatische oder aromatische Polyisocyanate oder deren Mischungen. Die Polyisocyanate sind bevozugt Diisocyanate, damit sich ein lineares Polymer ergibt, obwohl kleine Mengen an trifunktionellen Isocyanaten zusammen mit den Diisocyanaten eingesetzt werden können. Das Isocyanat liegt in einer Menge vor, die ausreicht, um mit den aktiven Wasserstoffatomen der 2,2-hydroxymethylsubstituierten Carbonsäure und der organischen Verbindungen, mit Ausnahme des Wasserstoffs an dem Carboxylat der 2,2-hydroxymethylsubstituierten Carbonsäure, zu reagieren. Diese Menge variiert, abhängig von der Menge an Carbonsäure und organischen Verbindungen.

Beispiele von Diisocyanaten umfassen, ohne jedoch darauf beschränkt zu sein, Methylendi-p-phenyldiisocyanat, Methylen-bis(4-cycohexylisocyanat), Isophorondiisocyanat, Toluoldiisocyanat, 1,5-Naphthalindiisocyanat, 4,4'-Diphenylmethandiisocyanat, 2,2'-Dimethyl-4,4'-diphenylmethandiisocyanat, 4,4'-Dibenzyldiisocyanat, 1,3-Phenylendiisocyanat, 1,4-Phenylendiisocyanat, Gemische von 2,4-und 2,6-Toluoldiisocyanat, 2,2'-Dichlor-4,4'-diphenylmethandiisocyanat, 2,4-Dibrom-1,5-naphthalindiisocyanat, Butan-1,4-diisocyanat, Hexan-1,6-diisocyanat, Cyclohexan-1,4-di-isocyanat.

Wenn man die Polymerkette nicht verlängern möchte, wird die Reaktion des Diisocyanats mit der organischen Verbindung, die zwei aktive Wasserstoffatome enthält, durch Zugabe einer monofunktionellen, aktiven Wasserstoff enthaltenden Verbindung abgebrochen, um so jegliche restliche Isocyanatfunktionalität zu verbrauchen. Beispiele dieser Kettenabbrecher sind auf diesem Fachgebiet wohlbekannt; für die vorliegenden Systeme ist der bevorzugte Kettenabbrecher Ethanol.

Die Polymerisation der Urethane wird im Reaktionsmedium mit oder ohne typische auf dem Fachgebiet bekannte Katalysatoren für die Urethanreaktion durchgeführt. Geeignete Katalysatoren umfassen Dibutylzinn-dilaurat, die Zinn(II)-salze von Carbonsäuren mit 2 bis 18 Kohlenstoffatomen, wie Zinn(II)-laurat, Zinn(II)-stearat, Zinn(II)-acetat, Zinn(II)-butyrat, Zinn(II)-octoat und dergleichen, sowie deren Mischungen. Zu anderen geeigneten Katalysatoren zählen Dibutylzinnoxid, Dibutylzinnsulfid, Bleiresinat,
Bleibenzoat, Bleisalicylat, Blei-2-ethylhexoat, Bleioleat, Eisenacetylacetonat, Kobaltbenzoat, Tetra (2-ethylhexyl)-titanat, Tetrabutyltitanat und dergleichen. Viele andere Verbindungen beschleunigen die Reaktion einer Hydroxyl- oder anderen Gruppe mit einem Isocyanat eher als gewisse andere Reaktionen der Isocyanatgruppe, und jede dieser Verbindungen kann verwendet werden. Die Fachleute werden einen speziellen Katalysator wählen, um den einzelnen Urethanreaktionen gewünschte charakteristische Merkmale zu verleihen. Die vorstehenden speziellen Verbindungen sind die bevorzugten Verbindungen und werden als erläuternd und nicht einschränkend genannt. Zusätzlich kann jedes geeignete tertiäre Amin, beispielsweise Triethylendiamin, N-Ethylmorpholin, N-Methylmorpholin oder 4-Dimethylamino-ethylpiperazin, entweder allein oder zusammen mit dem Metallkatalysator verwendet werden.

Was das Verhältnis der Reaktionsteilnehmer betrifft, so sollten sie so gewählt werden, dass das Molekularverhältnis von Isocyanatgruppen zu aktiven Wasserstoffatomen so nahe wie möglich bei 1 : 1 liegt. Sicherlich wird man dieses exakte Verhältnis in der Praxis nicht immer erreichen; daher sollte man ein Verhältnis von zwischen etwa 0,7 : 1 und 1,3 : 1 und vorzugsweise zwischen etwa 0,9 : 1 und 1,2 : 1 anstreben, und jeglicher Überschuss an Diisocyanat kann, wie zuvor besprochen, mit der monofunktionellen, aktiven Wasserstoff enthaltenden Verbindung unterdrückt werden.

Die Polymerisation wird nach bekannten Polymerisationsverfahren zur Herstellung von Polyurethan, die den Fachleuten gut bekannt sind, durchgeführt. Beispielhafte Polymerisationsverfahren und Reaktionsbedingungen sind in den Beispielen von DE69401230T2 angegeben.

Besonders bevorzugt ist die Verwendung des Haarpflegepolymers Polyurethan-14-AMP-Acrylat-Copolymer (DynamX^{®}).

Die vorstehend aufgezählten Haarpflegepolymere bilden auf der Haut kosmetisch unauffällige und flexible Filme. Kosmetisch unauffällige und flexible Filme liegen immer dann vor, wenn der gebildete Film transparent ist und die Haut kaum oder nur mäßig fixiert. Eine starke Hautfixierung würde zu unerwünschter Faltenbildung führen. Der Tragekomfort und die kosmetische Attraktivität wären gemindert. Die Filme zeigen während einer Tragezeit von mindestens 24 Stunden eine ausreichende Haftung (also kein Abblättern oder Ablösen) und weisen eine so hohe Flexibilität auf, dass sich während dieser Zeit keine sichtbaren Risse bilden. Eine längere Tragedauer von 72 - 84 Stunden - dies entspräche einer zwei mal wöchentlichen Anwendung - ist ebenfalls denkbar.

Die erfindungsgemäße pharmazeutische Zusammensetzung vereint dabei eine einfache Applikation und eine flexible Dosierung. Sie ist schnell trocknend, nicht klebrig und gut haftend. Die Wirkstofffreisetzung ist über einen längeren Zeitraum steuerbar. Für Hormone wird dabei eine höhere Wirkstoffpermeation durch die Humanepidermis als bei marktüblichen Hormon-Pflastern beziehungsweise aus ethanolischen Hormonlösungen gleicher Konzentration erreicht. Es erfolgt keine Kontamination der Kleidung oder anderer Personen.

Aus Viskositäts- und kosmetischen Gründen darf sich die Konzentration des filmbildenden Haarpflegepolymers in den pharmazeutischen Zubereitungen nur innerhalb bestimmter Grenzen bewegen. Ein zu hoher Polymeranteil führt zu einer deutlichen Viskositätserhöhung. Eine Applikation zum Beispiel durch Versprühen würde dadurch erschwert. Eine weitere Folge wäre die Bildung zu dicker Filme, die wiederum eine starke Fixierung der Haut und damit eine unerwünschte Faltenbildung verursachen würden. Die Dicke der so, durch Auftragung der pharmazeutischen Zubereitung auf die Haut, gebildeten Polymerenfilme liegt oberhalb von 1 µm und darf 100 µm nicht überschreiten. Bevorzugte Filmdicken liegen zwischen 5 µm und 50 µm.

Da die Menge des enthaltenen Polymers jedoch auch Einfluss auf die Wirkstoffbeladungskapazität hat, ist für jedes Polymer individuell ein Konzentrationsbereich festzulegen, der in Abhängigkeit von den Eigenschaften des Polymers und des Wirkstoffs einen Film erzeugt, der eine ausreichende Wirkstoffpermeation über den gewünschten Zeitraum ermöglicht und gleichzeitig den kosmetischen Anforderungen (keine Faltenbildung) entspricht. Die Polymerenkonzentration in der flüssigen pharmazeutischen Zubereitung (ohne Treibmittel) kann dabei Werte zwischen 0,01 bis 40 % (w/w), bevorzugt von 5 bis 30 % annehmen.

Die Polymerfilme zeigen darüber hinaus nur eine geringe Okklusivität, das heißt eine Wasserdampfdurchlässigkeit größer 0,05 g * cm⁻² * 24 h⁻¹ (Bestimmung gemäß Ph. Brit 1993 Appendix XXJ). Sie sind damit auch für längere Anwendungszeiten geeignet.

Die ebenfalls erfindungsgemäße pharmazeutische Zubereitung enthält neben einem oder mehreren der oben genannten Polymere mindestens ein geeignetes Lösungsmittel sowie mindestens einen Wirkstoff.

Als Wirkstoffe kommen alle arzneilich wirksamen Substanzen in Frage, die sich in die Polymerlösungen einarbeiten lassen und in entsprechenden Indikationen eine dermale bzw. transdermale Applikation erlauben. Auch die Applikation von Wirkstoffen zur Therapie dermaler Erkrankungen, wie zum Beispiel von Mykosen, mittels der erfindungsgemäßen pharmazeutischen Zubereitung ist denkbar. So können Hautläsionen gezielt, zum Beispiel durch Besprühen mit der Polymerlösung behandelt werden. Für die Behandlung verletzter Haut ist besonders auf die Verträglichkeit des Lösungsmittels zu achten.

Die folgende Aufzählung nennt Wirkstoffe, die zur Applikation mit den erfindungsgemäßen pharmazeutischen Zubereitungen geeignet sind:

Für die transdermale Applikation geeignet sind:
- Androgene, wie Testosteron und dessen Ester (wie zum Beispiel Testosterondipropionat), 7-Methly-11-fluor-19-nortestosteron oder 7-Methyl-19-nortestosteron
- Estrogene, wie Ethinylestradiol, Mestranol, Quinestranol, Estradiol, Estron, Estran, Estriol, Estetrol und konjugierte equine Estrogene,
- Gestagene, wie Progesteron, Hydroprogesteroncaproat, Levonorgestrel, Norgestimat, Norethisteron, Drospirenon, Dydrogesteron, Norelgestromin, Levonorgestrel, Dienogest, Lynestrenol, Etonogestrel, Medrogeston, Nestorone und Cyproteronacetat
- m-Cholinozeptor-Antagonisten, wie Scopolamin, Trospiumchlorid, Tiotropium und Homatropin
- Prostaglandine, wie Dinoprost, Misoprostol, Sulproston, und Gemeprost;
- sowie Danazol, Finasterid, Raloxifen, Nikotin, Oxytocin, Nitroglycerin, Fentanyl, Naloxon, Bupropion, Clonidin, Propranolol, Metoprolol, Diltiazem, Nicardipine, Captoril, Isosorbiddinitrat, Isosorbidmononitrat, Dimethylisosorbid, Talinolol, Lidocain, Propipocain, Diazepam, Midazolam, Methylphenidat, Parathormon, Rotigotine, Insulin, Heparin, Oxybutinin, Suphaguanidine und/oder Zidovudine,
inklusive ihrer chiralen Formen und pharmazeutisch akzeptablen Salze.

Für die dermale Applikation geeignet sind:
- die zu den Gerbstoffen zählenden Oligomere von Catechinen, Oligomere von Gallotanninen mit Flavanderivaten, Phenolcarbonsäuren, Gallussäure und deren Derivaten veresterte Zucker
- Antiseptika, wie Chlorhexidin, Triclosan und Ethacridin
- Antibiotika, wie Fusidinsäure, Erythromycin, Roxithromycin, Clarithromycin, Spiramycin, Minocylin, Clindamycin, Neomycin B, Kanamycin, Gentamycin, Amikacin, Tobramycin, Netilmicin Metronidazol, Nimorazol, Tinidazol Polymyxin B, Colistin, Tyrothricin, Bacitracin, Mupirocin und Cephalexin
- Antimykotika, wie Ketoconazol, Itraconazol, Amphotericin B, Griseofulvin, Fluconazol, Amorolfin, Flucytosin, Terbenafin, Naftifin, Ciclopirox, Natamycin, Nystatin, Undecylensäure und Isoconazol
- topische Kortikosteroide, wie Methylprednisolonaceponat, Clobetasolpropionat, Mometasonfuorat, Hydrocortison, Betamethason-17-benzoate, Prednicarbat, Diflucortolonvalerat, Triamcinilonacetonid, Amcinonid, Desoximetason, Fluocortolon und Fluticason
- topische Immunmodulatoren (Makrolide), wie Tacrolimus und Pimecrolimus, aber auch Epothilone
- die Antihistaminika Azelastin, Fexofenadin, Levocabastin, Loratadin, Mizolastin, Clemastin, Bamipin, Cetirizin, Dimetidin, Ketotifen und Emedastin
- die Immunsuppressiva Cyclosporin, Azathioprin und Mycophenolatmofetil,
- die Anthralinen Cignolin und Dithranol,
- die Vitamin D3-Analoga Calcipotriol und Tacalcitol
- die topischen Retinoide Tretinoin, Isotretinoin, Acitretin, Adapalen und Tazaroten
- die Zytostatika Methotrexat, 5-Fluorouracil, 5-Hydroxytamoxifen, Zink-Pyrithion und Fludarabin
- die UV-protektiven Stilbenderivate,
- die pflanzlichen Extrakte Grünteeextrakt, Centella asiatica Extrakt, Weidenrindenextrakt, Birkenextrakt, Teebaumöl, Olivenblattextrakt, Aloe vera Extrakt, Ringelblumenextrakt, Passionsblütenextrakt, Hamamelisextrakt, Kamillenextrakt, Bärentraubenblätterextrakt und Süßholzwurzelextrakt, beispielsweise als 18β-Glycyrhetinsäure (Zn Kombination) oder deren Mischungen, sowie
- Harnstoff, Milchsäure, Fumarsäureester, Azelainsäure, Hydrochinon, Benzoylperoxid, Benzylbenzoat, Ketoprofen, Ibuprofen, Salicylate, Naproxen, Diclofenac-Na und Salze, Ketorolac, Indomethacin, Piroxicam, Nicotinamid, Dipropylphthalate, Aminopyrin, Flufenaminsäure, Ketotifen, Polidocanol, Oligonukleotide, si-RNA und Ribozyme, RNA Decoy Nukleotide, Aciclovir, Penciclovir, Idoxuridin, Trifluridin, Vidarabin, Tromantadin, 5-Aminolävulinsäure, Lidocain, Procain und Cinchocain.

Der Wirkstoff kann dabei sowohl gelöst, als auch als Emulsion oder Suspension vorliegen.

Die Konzentration des Wirkstoffes in dem aus der pharmazeutischen Zubereitung entstehenden Polymerfilm richtet sich nach dem zu erzielenden Wirkstoffspiegel. Sie ist abhängig von der Hautgängigkeit des Wirkstoffes und gegebenenfalls von der Anwesenheit von Permeationsverbesserern in der Rezeptur. Bei Lösungen wird die einzuarbeitende Wirkstoffmenge begrenzt von der Sättigungslöslichkeit des Wirkstoffes in der Lösung. Wird diese überschritten, liegt der Wirkstoff teils gelöst und teils suspendiert vor. Die Menge der in der erfindungsgemäßen pharmazeutischen Zubereitung enthaltenden Wirkstoffe liegt vorzugsweise zwischen 0,01 und 15 % (w/w).

Die erfindungsgemäßen Polymerfilme sind sehr dünne Systeme mit begrenzter Beladungskapazität. Bevorzugt eignen sich daher Wirkstoffe zur Applikation durch dieses System, die entweder strukturell bedingt eine gute Hautgängigkeit haben, durch das Polymersystem an sich oder einen permeationsfördernden Zusatz sehr effizient durch die Haut transportiert werden können oder hochpotent sind und daher nur in geringer Dosis appliziert werden müssen. Besonders geeignet sind hierbei Hormone, vor allem Estrogene, Gestagene und Androgene, speziell Ethinylestradiol und Levonorgestrel.

Um lange Wartezeiten bei der Trocknung der Zubereitung zu vermeiden, die die Patienten-Compliance beeinträchtigen können, werden hautverträgliche, leicht flüchtige Lösungsmittel eingesetzt. Bevorzugt sind dies Ethanol, Isopropanol, Ethylacetat, flüchtige Silicone, Aceton und Wasser. Die Lösungsmittel können allein oder in Kombination miteinander eingesetzt werden. Bei wasserhaltigen Lösungsmittelgemischen liegt der Wasseranteil aufgrund der ansonsten verlängerten Trocknungsdauer vorzugsweise unter 50 % (w/w).

Gegebenenfalls kann den pharmazeutischen Zubereitungen ein oder mehrere Weichmacher zugesetzt werden. Bevorzugte Weichmacher sind Triethylcitrat, Tributylcitrat, Acetyltriethylcitrat, Acetyltributylcitrat, Triacetin, Dibutylphthalat, Tributylsebacat, Diethylphtalat, Propylenglycol, Polyethylenglycol, Glycerin oder Rhizinusöl. Der Weichmachergehalt muss dabei auf das Polymer und seine Konzentration abgestimmt sein, da bei zu hohem Weichmachergehalt der Film klebrig wird. Bei zu geringem Weichmachergehalt hingegen sinkt die Adhäsivität und Flexibilität. Der Film würde dann nach einer gewissen Zeit abblättern oder rissig werden. Der Anteil an gegebenenfalls zuzusetzendem Weichmacher in der erfindungsgemäßen pharmazeutischen Zusammensetzung beträgt vorzugsweise zwischen 0,01 und 20 % (w/w). Die Weichmacher können allein oder in Kombination miteinander eingesetzt werden.

Daneben können gegebenenfalls noch weitere pharmazeutisch verträgliche Stoffe wie Feuchthaltemittel und Emulgatoren den erfindungsgemäßen pharmazeutischen Zusammensetzungen zugesetzt werden.

Als Feuchthaltemittel kommen bevorzugt Glycerol, Sorbitol, Propylenglycol, Polyethylenglycol oder Polyvinylpyrrolidon sowie deren Kombinationen in Frage. Ihr Anteil an der pharmazeutischen Zubereitung liegt bevorzugt zwischen 0,01 und 10 % (w/w).

Erfindungsgemäß bevorzugte Emulgatoren sind Na-Cetylstearylsulfat, Glycerolfettsäureester, Lecithin, Fettalkohole, Cholesterol, Sorbitanfettsäureester, Polyoxyethylen(POE)-Fettsäureester, POE-Fettsäureglyceride oder POE-Fettalkoholether. Diese können der pharmazeutischen Zubereitung bevorzugt mit einem Anteil zwischen 0,01 und 10 % (w/w) zugesetzt werden.

Eine Steuerung des Wirkstofftransports aus dem Polymersystem über die Haut kann sowohl über die Auswahl eines speziellen permeationsfördernden Stoffes oder einer Kombination verschiedener Stoffe erfolgen als auch über die Menge des bzw. der zugesetzten Stoffe.

Gegebenenfalls zuzusetzende bevorzugte Permeationsverbesserer sind ausgewählt aus der Gruppe der Laurocaprame, Sulfoxide, Terpene oder ätherischen Öle, Ölsäure, Oleylalkohol, Laurylsäure, Propylenglycol, Propylencarbonat, N-Methyl-Pyrrolidon oder Isopropylmyristat. Die Permeationsförderer können dabei sowohl einzeln als auch in Kombinationen eingesetzt werden. Besonders bevorzugt sind Ölsäure, das Terpen R-(+)-Limonen und Isopropylmyristat. Ganz besonders bevorzugt sind Isopropylmyristat mono und Kombinationen von Ölsäure, R-(+)-Limonen oder Isopropylmyristat mit Propylenglycol, bevorzugt im Verhältnis 1:1.

Besonders vorteilhaft für den Wirkstoff Ethinylestradiol sind beispielsweise R-(+)-Limonen, Ölsäure und Isopropylmyristat, vor allem in Kombination mit Propylenglycol. Ihr Anteil an der pharmazeutischen Zubereitung liegt bevorzugt zwischen 0,01 und 15 % (w/w).

Neben den Permeationsverbesserern können auch die Grundlösungsmittel wie zum Beispiel Ethanol, Ethylacetat oder Isopropanol zur Permeationsförderung beitragen. Jedoch ist die Wirkstoffpermeation aus einem, aus einer ethanolischen Polymerlösung entstandenen Polymerfilm derjenigen aus einer ethanolischen Wirkstofflösung ohne Polymer deutlich überlegen. Die Anwesenheit des organischen Lösungsmittels allein bedingt also nicht die erhöhte Permeation des Wirkstoffes durch Humanepidermis. Das Polymer spielt bei diesem Vorgang eine wichtige Rolle. Diese Beobachtung wird auch dadurch gestützt, dass aus Zubereitungen mit unterschiedlichen Polymeren unterschiedliche Mengen an Wirkstoff durch Humanepidermis transportiert werden.

Bei Einarbeitung größerer Wirkstoffmengen oder bei Zusatz pharmazeutischer Hilfsstoffe in größerem Umfang ist die Rezeptur zum Beispiel durch Erhöhung des Weichmachergehalts anzupassen, da sich die Filmeigenschaften zum Beispiel hinsichtlich der Haftfähigkeit durch die zugesetzten Stoffe ändern können.

Die erfindungsgemäßen pharmazeutischen Zubereitungen können in handelsübliche Applikatoren abgefüllt und mit ihrer Hilfe auf die Haut aufgetragen werden. Insbesondere geeignet sind hierzu Roller, Pumpsprayflaschen, Spraydosen, Tuben, Pinselflaschen oder Pipettenflaschen. Im Falle der Abfüllung in eine Spraydose bedarf es der zusätzlichen Beimischung eines Treibmittels. Als Treibmittel kann zum Beispiel Dimethylether verwendet werden. Die Dosierung der Wirkstoffe erfolgt dabei mittels dem Fachmann bekannter Vorrichtungen beziehungsweise Verfahren.

Für Ethinylestradiol und Levonorgestrel konnte gezeigt werden, dass sich mit den neuen pharmazeutischen Zubereitungen sowohl mit als auch ohne Permeationverbesserer mehr Wirkstoff durch die Human-Epidermis transportieren lässt als aus einer ethanolischen Lösung gleicher Konzentration. Die neuen Polymersysteme sind einfachen ethanolischen Lösungen gleicher Wirkstoffkonzentration im Hinblick auf die Menge an innerhalb von 24 Stunden permeiertem Wirkstoff überlegen.

Aus der erfindungsgemäßen pharmazeutischen Zubereitung konnte mit und ohne Zusatz von Permeationsförderern innerhalb von 24 Stunden signifikant mehr Ethinylestradiol pro Flächeneinheit durch die Human-Epidermis transportiert werden als aus einem handelsüblichen Kontrazeptionspflaster (EVRA^{®}; Wirkstoffe: Ethinylestradiol und Norelgestromin).

### Beispiel 1: pharmazeutische Zubereitung ohne Permeationsverbesserer

Um eine pharmazeutische Zubereitung ohne Permeationsverbesserer zu finden, die auf der Haut zu flexiblen, kosmetisch unauffälligen Filmen trocknet, wurde ein zu applizierender Wirkstoff gegebenenfalls unter Erwärmen in der Regel in 96%igem Ethanol gelöst. Nach der Auflösung des Wirkstoffs wurde ein Polymer zugegeben und die Zubereitung bis zur vollständigen Auflösung des Polymers auf dem Magnetrührer gerührt. Nach Erhalt einer klaren Lösung wurden gegebenenfalls Quervernetzer oder Weichmacher zugegeben und die Lösung für weitere 24 Stunden gerührt. Die Aufbewahrung der Polymerenlösung erfolgte in Glasgefäßen, die dicht mit einem silikonisierten Gummistopfen und einer Aluminium-Bördelkappe verschlossen waren. Polymer, Polymergehalt, Weichmacher und Weichmachergehalt wurden bei diesen Formulierungsversuchen variiert.

Die so erhaltenen Zubereitungen wurden anhand der Kriterien Viskosität, Trocknungszeit, Klebrigkeit der Außenseite, kosmetische Attraktivität und Nachhaltigkeit auf der Haut wie nachfolgend beschrieben beurteilt. Dazu wurden etwa 50 mg (entsprechend 10 mg/cm²) der so erhaltenen Zubereitungen mit Hilfe einer Metallschablone auf die Haut aufgetragen.

Fünf Minuten nach dem Auftragen der Zubereitung auf die Haut wurde die Trocknung des Films durch druckloses Auflegen eines Objektträgers aus Glas auf den Film geprüft. Der Film wurde als trocken angesehen, wenn auf dem Objektträger nach dem Abheben vom Film keine Flüssigkeitsreste erkennbar waren. Um eine gute Patientencompliance zu gewährleisten sind nur Filme, die nach 5 Minuten als trocken anzusehen sind, zu selektieren.

Zur Prüfung der Klebrigkeit der Filmaußenseite wurde ein Wattebausch auf den trockenen Film unter geringem Druck aufgebracht. Die Beurteilung erfolgte anhand der Menge der vom Film zurückgehaltenen Watte-Fasern.

Die kosmetische Attraktivität des Films wurde visuell mit dem bloßen Auge im Hinblick auf Dicke, Transparenz und Grad der Hautfixierung des Films bewertet.

Die Nachhaltigkeit des Films auf der Haut wurde nach einer Tragezeit von 24 Stunden visuell mit einer Lupe mit 10facher Vergrößerung beurteilt. Je weniger Risse und Lücken der Film (Abblättern) aufwies, um so besser war seine Qualität.

Die Viskosität der Lösungen wurde im Aufbewahrungsgefäß visuell mit dem Ziel beurteilt, niedrig bis mittelviskose Zubereitungen zu selektieren, die durch Versprühen appliziert werden können.

Eine Zubereitung wurde dann als erfolgreich angesehen, wenn in allen der vorgenannten Kriterien die beste Bewertung (niedrige bis mittlere Viskosität, Trocknung nach 5 Minuten, keine bis allenfalls geringe Klebrigkeit der Filmaußenseite, geringe Dicke, gute Transparenz und nur geringe Hautfixierung sowie geringe oder keine Riss- oder Lückenbildung nach 24 Stunden) erzielt wurde. Jede Änderung der erfindungsgemäßen pharmazeutischen Zubereitungen hinsichtlich ihrer Inhaltsstoffe und deren Konzentration hat eine Änderung der Filmeigenschaften zur Folge, die dazu führen kann, dass sich die Bewertung der genannten Kriterien ändert. Innerhalb gewisser Grenzen sind diese Änderungen akzeptabel, so dass Konzentrationsspannen für die einzelnen Bestandteile angegeben werden können. Starke Abweichungen jedoch führen zu Polymerfilmen auf der Haut, die nicht mehr die beschriebenen gewünschten Eigenschaften aufweisen und somit für die geplante Anwendung nicht mehr geeignet sind.

Die nachfolgende Tabelle enthält Angaben über die Mengenbereiche innerhalb derer die pharmazeutischen Zubereitungen eine flexiblen, gut haftenden, nicht klebrigen und kosmetisch unauffälligen Film auf der Haut bilden. Einen besonders geeigneten Film bildet eine pharmazeutischen Zusammensetzung aus 10 % (w/w) DynamX®, 1 % (w/w) Triethylcitrat, 89 % (w/w) Ethanol und 5 % (w/w) Wirkstoff.

| | Mengenbereich | Beispiel 1 |
|---|---|---|
| DynamX^{®} | 0,1 - 40 % (w/w) | 10 % (w/w) |
| Triethylcitrat | 0,1 - 18 % (w/w) | 1 % (w/w) |
| Ethanol | 32 - 99,7 % (w/w) | 89 % (w/w) |
| Wirkstoff | 0,1 - 10 % (w/w) | 5 % (w/w) |

### Beispiel 2: pharmazeutische Zubereitung mit Permeationsverbesserer

Um den Transport des Wirkstoffs durch die Haut zu erhöhen, können den hinsichtlich der obengenannten Kriterien positiv beurteilten Formulierungen permeationsfördernde Stoffe zugesetzt werden.

| | Mengenbereich | A | B | C | D | E | F |
|---|---|---|---|---|---|---|---|
| DynamX^{®} | 0,1 - 40% | 10% | 10% | 10% | 10% | 10% | 10% |
| Triethylcitrat | 0,1 - 18% | 1 % | 1 % | 1 % | 1 % | 1 % | 1 % |
| Ölsäure | 0,1 - 10% | 5% | 2,5% | 1,5% | | | |
| R-(+)-Limonen | 0,1 - 10% | | | | 5 % | 2,5% | |
| Isopropylmyristat | 0,1 - 10% | | | | | | 5% |
| Propylenglycol | 0,1 - 10% | | 2,5% | 1,5% | | 2,5% | |
| Ethanol | 22 - 99,8% | 79% | 79% | 81 % | 79% | 79% | 79% |
| Wirkstoff | 0,1 - 10% | 5% | 5% | 5% | 5% | 5% | 5% |

| | | | | | | | |
|---|---|---|---|---|---|---|---|
| %-Angaben meinen % (w/w) | | | | | | | |

### Beispiel 3: Permeationsverqleich mit ethanolischer Lösung

Für die Hautpermeationsversuche wurde humane Bauchhaut aus plastischen Operationen verwendet. Die Haut wurde vom unterliegenden Fettgewebe befreit und bis zur Verwendung eingefroren aufbewahrt. Zur Präparation wurden runde Proben aus den Hautstücken ausgestanzt und die Epidermis durch Hitzseparierung von den übrigen Hautbestandteilen getrennt (Kligman, A.M., Christophers, E., Preparation of isolated sheets of human stratum corneum. Archives of Dermatology, 1963. 88: p. 702-705). Die Epidermis wurde zusammen mit einer geeigneten Stützmembran in vertikale Franz-Diffusionszellen eingespannt. Als Akzeptormedium wurde neutraler Phosphatpuffer mit 0,5% Y-Cyclodextrin als Lösungsvermittler eingesetzt. Während des gesamten Versuchs wurden die Zellen auf 32°C temperiert und das Akzeptormedium in den Zellen mit einem Magnetrührstab kontinuierlich durchmischt. Nach Auftragen der jeweiligen Formulierung auf die Epidermis (jeweils vier Zellen pro Formulierung) wurde zu definierten Zeitpunkten aus jeder Zelle eine Probe entnommen und das Volumen durch frisches Akzeptormedium ersetzt. Der Wirkstoffgehalt der Proben wurde ohne weitere Aufarbeitung per HPLC bestimmt.

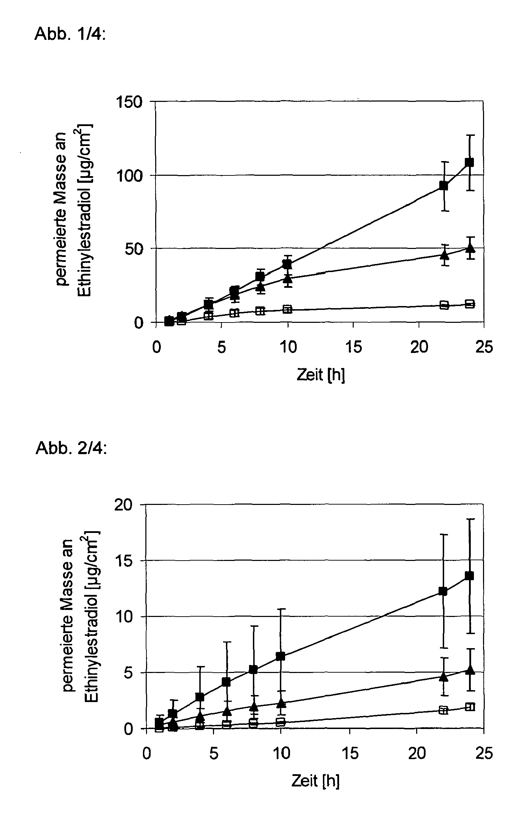

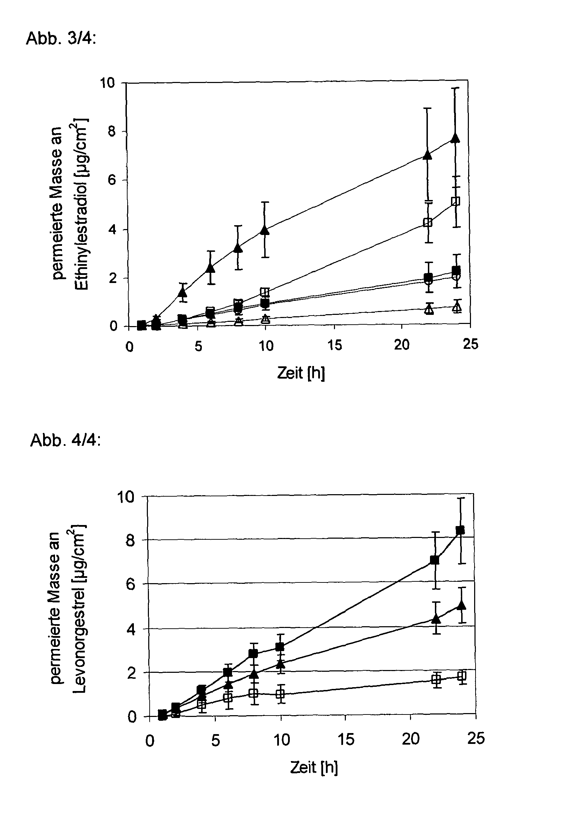

Es wurde die Permeation von Ethinylestradiol aus dem erfindungsgemäßen Polymersystem (DynamX 10 % (w/w); Ethinylestradiolgehalt 5 % (w/w)) mit und ohne Permeationsverbesserer (Ölsäure 2,5 % (w/w); Propylenglycol 2,5 %, (w/w)) durch eine hitzeseparierte Humanepidermis bestimmt und mit der Permeation aus einer ethanolischen Lösung (5 % (w/w) Ethinylestradiol) verglichen. Die Ergebnisse der Permeationsversuche sind in Abbildung 1/4 grafisch dargestellt.

### Beispiel 4: Permeationsveraleich mit Pflaster

Es wurde die Permeation von Ethinylestradiol aus dem erfindungsgemäßen Polymersystem (DynamX 10 % (w/w); Ethinylestradiolgehalt 5 % (w/w)) mit und ohne Enhancer (Ölsäure 2,5 % (w/w); Propylenglycol 2,5 %, (w/w)) durch eine hitzeseparierte Humanepidermis bestimmt und mit der Permeation von Ethinylestradiol aus einem handelsüblichen, kontrazeptiven Pflaster (EVRA^{®}) verglichen. Die Ergebnisse der Permeationsversuche sind in Abbildung 2 grafisch dargestellt.

### Beispiel 5: Permeationsvergleich von verschiedenen Polymerlösungen

Es wurde die Permeation von Ethinylestradiol aus dem erfindungsgemäßen Polymersystem (DynamX 10 % (w/w); Ethinylestradiolgehalt 5 % (w/w)) durch eine hitzeseparierte Humanepidermis bestimmt und mit der Permeation von Ethinylestradiol aus Polymerlösungen mit anderen Polymeren und gleichem Ethinylestradiol-Gehalt (5 % (w/w)) und mit einer ethanolischen Lösung (5 % (w/w) Ethinylestradiol) verglichen. Die Ergebnisse der Permeationsversuche sind in Abbildung 3 grafisch dargestellt.

### Beispiel 6: Levonorgestrel

Es wurde die Permeation von Levonorgestrel aus dem erfindungsgemäßen Polymersystem (DynamX 10 %; Levonorgestrelgehalt 0,3 % (w/w)) mit und ohne Enhancer (Ölsäure 2,5 %; Propylenglycol 2,5 %, (w/w)) durch eine hitzeseparierte Humanepidermis bestimmt und mit der Permeation von Levonorgestrel aus einer ethanolischen Lösung (0,3 % w/w Levonorgestrel) verglichen. Die Ergebnisse der Permeationsversuche sind in Abbildung 4 grafisch dargestellt.

### Abbildungsverzeichnis:

Abbildung 1/4 [Beispiel 3] zeigt die Ergebnisse der Vergleichsversuche zur Wirkstoffpermeation (Ethinylestradiol) aus der erfindungsgemäßen pharmazeutischen Zubereitung mit (geschlossene Quadrate) und ohne (geschlossene Dreiecke) Permeationsverbesserer und aus einer ethanolischen Lösung (offene Quadrate).

Abbildung 2/4 [Beispiel 4] zeigt die Ergebnisse der Vergleichsversuche zur Wirkstoffpermeation (Ethinylestradiol) aus der erfindungsgemäßen pharmazeutischen Zubereitung mit (geschlossene Quadrate) und ohne (geschlossene Dreiecke) Permeationsverbesserer und aus einem kontrazeptiven Pflaster (offene Quadrate).

Abbildung 3/4 [Beispiel 5] zeigt die Ergebnisse der Vergleichsversuche zur Wirkstoffpermeation (Ethinylestradiol) aus der erfindungsgemäßen pharmazeutischen Zubereitung mit dem Polymer DynamX (geschlossene Dreiecke) und aus Polymersystemen mit anderen Polymeren wie Klucel LF (=Hydroxypropylcellulose, offene Quadrate), SGM 36 (=Silicongummi, geschlossene Quadrate) und Eudragit RL PO (= Ammoniummethacrylat Copolymer, offene Dreiecke) sowie aus einer ethanolischen Lösung (offene Kreise).

Abbildung 4/4 [Beispiel 6] zeigt die Ergebnisse der Vergleichsversuche zur Wirkstoffpermeation (Levonorgestrel) aus der erfindungsgemäßen pharmazeutischen Zubereitung mit (geschlossene Quadrate) und ohne (geschlossene Dreiecke) Permeationsverbesserer und aus einer ethanolischen Lösung (offene Quadrate).

## Patentansprüche

1. Verwendung eines filmbildenden in Haarpflegemitteln Verwendung findenden Polymeres oder eines Gemisches verschiedener dieser Polymere in pharmazeutischen Zubereitungen zur dermalen oder transdermalen Applikation von Wirkstoffen.

2. Verwendung nach Anspruch 1, **dadurch gekennzeichnet, dass** das Haarpflegepolymer ausgewählt ist aus der Gruppe:
o Polyurethane,
o Monoalkylester von Poly(methylvinylether/Maleinsäureanhydrid bzw. Maleinsäure),
o Acrylat/Alkylacrylamid Copolymere,
o Acrylat/Alkylacrylat/Ethylaminoxid Methacrylat Copolymere,
o Acrylat/Itaconat Copolymere,
o Vinylacetat/Crotonat Copolymere,
o Vinylcaprolactam/Vinylpyrrolidon/Methacrylat Copolymere oder
o Vinylpyrrolidon/Methacrylamid Copolymere.

3. Verwendung nach Anspruch 2, **dadurch gekennzeichnet, dass** die Haarpflegepolymere ausgewählt sind aus der Gruppe:
o Polyurethane-1,
o Polyurethane-14 und AMP-Acrylat Copolymere,
o Polyurethane-2,
o Polyurethane-4,
o Polypropylenglycol-17/ Isophoronediisocyanat/ Dimethylpropionsäure Copolymer,
o Monoethylester des Methylvinylether und Maleinsäureanhydrid Copolymers
o Ethylester des Polyvinylmethylether/Maleinsäureanhydrid Copolymers
o Isopropylester des Polyvinylmethylether/Maleinsäureanhydrid Copolymers
o Butylester des Polyvinylmethylether/ Maleinsäureanhydrid Copolymers
o Octylacrylamid/Acrylat/Butylaminoethylmethacrylat Copolymer,
o Acrylat/Octylacrylamid Copolymer,
o Acrylat/Ethylacrylat/N-tert-butylacrylamid Copolymer,
o Acrylat/Laurylacrylat/Stearylacrylat/Ethylaminoxid Methacrylat Copolymer,
o Acrylat/Stearylacrylat/Ethylaminoxid Methacrylat Copolymer,
o Acrylat/Steareth-20 Itaconat Copolymer
o Acrylat/Ceteth-20 Itaconat Copolymer
o Acrylat/Aminoacrylat/C10-30 Alkyl PEG-20 Itaconat Copolymer,
o Vinylacetat/Crotonat Copolymer,
o Vinylacetat/Crotonat/Vinylneodecanoat Copolymer,
o Vinylpyrrolidon/Methacrylamid/Vinylimidazol Copolymer,
o VinylcaprolactamNinylpyrrolidon/Dimethylaminoethylmethacrylat Copolymer,
o VinylcaprolactamNinylpyrrolidon/Dimethylaminoethylmethacrylat Copolymer und Laurylpyrrolidon,
o Vinylpyrrolidon/Vinylcaprolactam/Dimethylaminopropylmethacrylamid Copolymer,
o Vinylpyrrolidon/Dimethylaminopropylmethacrylamid Copolymer,
o Vinylpyrrolidon/Dimethylaminopropylmethacrylamid/Methacryloylaminoprop yl lauryl dimethyl ammonium chlorid Copolymer
o Vinylpyrrolidon/Methacrylamidopropyl trimethylammonium chlorid Copolymer.

4. Verwendung nach Anspruch 1 **dadurch gekennzeichnet, dass** das Haarpflegepolymer ein gegebenenfalls neutralisiertes vollständig ausreagiertes carboxyliertes lineares Polyurethan, umfassend das Reaktionsprodukt aus
(1) einer oder mehreren 2,2-hydroxymethylsubstituierten Carbonsäure(n), dargestellt durch die Formel I in der R Wasserstoff oder C₁-C₂₀-Alkyl bedeutet, vorhanden in einer Gewichtsmenge, die ausreicht, um 0,35 - 2,25 Milliäquivalent Carboxylfunktionalität pro Gramm Polyurethan zu ergeben, 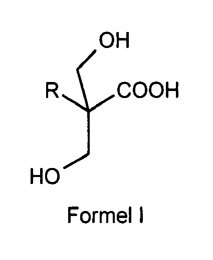
(2) 10 - 90 Gewichts-%, bezogen auf das Gewicht des Polyurethans, einer oder mehrerer organischen Verbindung(en), die jeweils nicht mehr als zwei aktive Wasserstoffatome aufweisen, und
(3) einem oder mehreren organischen Diisocyanat(en), vorhanden in einer Menge, die ausreicht, um mit den aktiven Wasserstoffatomen der 2,2-hydroxymethylsubstituierten Carbonsäure und der organischen Verbindungen, mit Ausnahme des Wasserstoffs an dem Carboxylat der 2,2-hydroxymethylsubstituierten Carbonsäure, zu reagieren;
ist.

5. Verwendung nach Anspruch 5, **dadurch gekennzeichnet, dass** das Haarpflegepolymer Polyurethan-14-AMP-acrylat-Copolymer (DynamX^{®}) ist.

6. Verwendung nach einem der Ansprüche 1 bis 5 **dadurch gekennzeichnet, dass** die Polymerkonzentration in der Lösung 0,01 - 40 %, bevorzugt 5 - 30 % beträgt.

7. Flüssige pharmazeutische Zubereitung, enthaltend neben ein oder mehreren der in den Ansprüchen 1 bis 6 genannten Haarpflegepolymeren:
o 0,01 - 15 % (w/w) eines oder mehrerer Wirkstoffe,
o 0,01 - 99,98 % (w/w) eines oder mehrerer Lösungsmittel,
o gegebenenfalls 0,01 - 20 % (w/w) eines oder mehrerer Weichmacher,
o gegebenenfalls 0,01 - 10 % (w/w) eines oder mehrerer Feuchthaltemittel,
o gegebenenfalls 0,01 - 10 % (w/w) eines oder mehrerer Emulgatoren und/oder
o gegebenenfalls 0,01 - 15 % (w/w) eines oder mehrerer Permeationsverbesserer.

8. Flüssige pharmazeutische Zubereitung nach Anspruch 7 in Form einer Lösung, Emulsion oder Suspension.

9. Flüssige pharmazeutische Zubereitung nach Anspruch 7 oder 8 zur transdermalen Applikation eines Wirkstoffes.

10. Flüssige pharmazeutische Zubereitung nach Anspruch 9 enthaltend einen oder mehrere Wirkstoffe ausgewählt aus der Gruppe der:
o der Androgene Testosteron und dessen Ester (wie zum Beispiel Testosterondipropionat), 7-Methly-11-fluor-19-nortestosteron oder 7-Methyl-19-nortestosteron
o die Estrogene Ethinylestradiol, Mestranol, Quinestranol, Estradiol, Estron, Estran, Estriol, Estetrol und konjugierte equine Estrogene,
o der Gestagene Progesteron, Hydroprogesteroncaproat, Levonorgestrel, Norgestimat, Norethisteron, Drospirenon, Dydrogesteron, Norelgestromin, Levonorgestrel, Dienogest, Danazol, Lynestrenol, Etonogestrel, Medrogeston, Nestorone und Cyproteronacetat
o der m-Cholinozeptor-Antagonisten Scopolamin, Trospiumchlorid, Tiotropium und Homatropin
o der Prostaglandine Dinoprost, Misoprostol, Sulproston, und Gemeprost;
o sowie Finasterid, Raloxifen, Nikotin, Oxytocin, Nitroglycerin, Fentanyl, Naloxon, Bupropion, Clonidin, Propranolol, Metoprolol, Diltiazem, Nicardipine, Captoril, Isosorbiddinitrat, Dimethylisosorbid, Talinolol, Lidocain, Propipocain, Diazepam, Midazolam, Methylphenidat, Parathormon, Rotigotine, Insulin, Heparin, Oxybutinin, Suphaguanidine und/oder Zidovudine,
inklusive ihrer chiralen Formen und pharmazeutisch akzeptablen Salze.

11. Flüssige pharmazeutische Zubereitung nach Anspruch 7 oder 8 zur dermalen Applikation eines Wirkstoffes.

12. Flüssige pharmazeutische Zubereitung nach Anspruch 11 enthaltend einen oder mehrere Wirkstoffe ausgewählt aus der Gruppe:
o der zu den Gerbstoffen zählenden Oligomere von Catechinen, Oligomere von Gallotanninen mit Flavanderivaten, Phenolcarbonsäuren, Gallussäure und deren Derivaten veresterte Zucker
o der Antiseptika Chlorhexidin, Triclosan und Ethacridin
o der Antibiotika Fusidinsäure, Erythromycin, Roxithromycin, Clarithromycin, Spiramycin, Minocylin, Clindamycin, Neomycin B, Kanamycin, Gentamycin, Amikacin, Tobramycin, Netilmicin Metronidazol, Nimorazol, Tinidazol Polymyxin B, Colistin, Tyrothricin, Bacitracin, Mupirocin und Cephalexin
o der Antimykotika Ketoconazol, Itraconazol, Amphotericin B, Griseofulvin, Fluconazol, Amorolfin, Flucytosin, Terbenafin, Naftifin, Ciclopirox, Natamycin, Nystatin, Undecylensäure und Isoconazol
o der topischen Kortikosteroide Methylprednisolonaceponat, Clobetasolpropionat, Mometasonfuorat, Hydrocortison, Betamethason-17-benzoate, Prednicarbat, Diflucortolonvalerat, Triamcinilonacetonid, Amcinonid, Desoximetason, Fluocortolon und Fluticason
o der topischen Immunmodulatoren (Makrolide) Tacrolimus und Pimecrolimus,
o der Antihistaminika Azelastin, Fexofenadin, Levocabastin, Loratadin, Mizolastin, Clemastin, Bamipin, Cetirizin, Dimetidin, Ketotifen und Emedastin
o der Immunsuppressiva Cyclosporin, Azathioprin und Mycophenolatmofetil,
o der Anthralinen Cignolin und Dithranol,
o der Vitamin D3-Analoga Calcipotriol und Tacalcitol
o der topischen Retinoide Tretinoin, lsotretinoin, Acitretin, Adapalen und Tazaroten
o der Zytostatika Methotrexat, 5-Fluorouracil, 5-Hydroxytamoxifen, Zink-Pyrithion und Fludarabin
o der UV-protektiven Stilbenderivate,
o der pflanzlichen Extrakte Grünteeextrakt, Centella asiatica Extrakt, Weidenrindenextrakt, Birkenextrakt, Teebaumöl, Olivenblattextrakt, Aloe vera Extrakt, Ringelblumenextrakt, Passionsblütenextrakt, Hamamelisextrakt, Kamillenextrakt, Bärentraubenblätterextrakt und Süßholzwurzelextrakt, beispielsweise als 18β-Glycyrhetinsäure (Zn Kombination) oder deren Mischungen, sowie
o Harnstoff, Milchsäure, Fumarsäureester, Azelainsäure, Hydrochinon, Benzoylperoxid, Benzylbenzoat, Ketoprofen, Ibuprofen, Salicylate, Naproxen, Diclofenac-Na und Salze, Ketorolac, Indomethacin, Piroxicam, Nicotinamid, Dipropylphthalate, Aminopyrin, Flufenaminsäure, Ketotifen, Polidocanol, Oligonukleotide, si-RNA und Ribozyme, RNA Decoy Nukleotide, Aciclovir, Penciclovir, Idoxuridin, Trifluridin, Vidarabin, Tromantadin, 5-Aminolävulinsäure, Lidocain, Procain und Cinchocain.

13. Flüssige pharmazeutische Zubereitung nach einem der Ansprüche 7 bis 12 **dadurch gekennzeichnet, dass** das Lösungsmittel hautverträglich und flüchtig ist.

14. Flüssige pharmazeutische Zubereitung nach Anspruch 13 enthaltend ein oder mehrere Lösungsmittel ausgewählt aus der Gruppe:
o Ethanol,
o Isopropanol,
o Ethylacetat,
o flüchtige Silikone,
o Aceton oder
o Wasser.

15. Flüssige pharmazeutische Zubereitung nach Anspruch 13 oder 14 wobei das Lösungsmittel ein wässriges Lösungsmittelgemisch ist dessen Wasseranteil unter 50 % (w/w) liegt.

16. Flüssige pharmazeutische Zubereitung nach einem der Ansprüche 7 bis 12, enthaltend einen oder mehrere Weichmacher ausgewählt aus der Gruppe:
o Triethylcitrat,
o Tributylcitrat,
o Acetyltriethylcitrat,
o Acetyltributylcitrat,
o Triacetin,
o Dibutylphtalat,
o Dibutylsebacat,
o Diethylphthalat,
o Propylenglycol,
o Polyethylenglycol,
o Glycerin oder
o Rhizinusöl.

17. Flüssige pharmazeutische Zubereitung nach einem der Ansprüche 7 bis 12, enthaltend ein oder mehrere Feuchthaltemittel, ausgewählt aus der Gruppe:
o Glycerol,
o Sorbitol,
o Propylenglycol,
o Polyethylenglycol oder
o Polyvinylpyrrolidon.

18. Flüssige pharmazeutische Zubereitung nach einem der Ansprüche 7 bis 12, enthaltend einen oder mehrere Emulgatoren ausgewählt aus der Gruppe:
o Na-Laurylsulfat,
o Na-Cetylstearylsulfat,
o Glycerolfettsäureester,
o Lecithin,
o Fettalkohole,
o Cholesterol,
o Sorbitanfettsäureester,
o Polyoxyethylen-Fettsäureester,
o Polyoxyethylen-Fettsäureglyceride oder
o Polyoxyethylen-Fettalkoholether.

19. Flüssige pharmazeutische Zubereitung nach einem der Ansprüche 7 bis 12, enthaltend einen oder mehrere Permeationsverbesserer ausgewählt aus der Gruppe:
o Laurocaprame,
o Sulfoxide,
o Terpene oder ätherisches Öl,
o Ölsäure,
o Oleylalkohol,
o Laurylsäure,
o Propylenglycol,
o Propylencarbonat,
o N-Methyl-Pyrrolidon oder
o Isopropylmyristat.

20. Pharmazeutische Zubereitung nach Anspruch 19, enthaltend einen oder mehrere Permeationsverbesserer ausgewählt aus der Gruppe:
o Ölsäure,
o R-(+)-Limonen,
o Isopropylmyristat, insbesondere Isopropylmyristat mono,
o Kombination von Ölsäure mit Propylenglycol, bevorzugt im Verhältnis 1:1 oder
o R-(+)-Limonen mit Propylenglycol, bevorzugt im Verhältnis 1:1 oder
o Isopropylmyristat mit Propylenglycol.

21. Flüssige pharmazeutische Zubereitung nach einem der Ansprüche 7 bis 20 **dadurch gekennzeichnet, dass** diese sprühfähig ist und gegebenenfalls zusätzlich ein Treibmittel enthält.

22. Applikator enthaltend eine flüssige pharmazeutische Zubereitung nach einem der Ansprüche 7 bis 22, insbesondere in Form eines Rollers, einer Pumpspraydose, einer Spraydose, einer Tube, einer Pinselflasche oder einer Pipettenflasche.

23. Flexibles, gut haftendes, nicht klebriges, kosmetisch unauffälliges Pflaster zur dermalen oder transdermalen Applikation eines Wirkstoffes erhältlich durch Auftragen einer pharmazeutischen Zubereitung gemäß einem der Ansprüche 7 bis 21 auf die Haut und anschließender Verdunstung des Lösungsmittels.

24. Pflaster nach Anspruch 23, **dadurch gekennzeichnet, dass** die Wasserdampfdurchlässigkeit über 0,05g*cm⁻²*24h⁻¹ liegt.

25. Pflaster nach Anspruch 23 enthaltend das Haarpflegepolymer DynamX^{®}.
